(19) Europäisches Patentamt
European Patent Office
Office européen des brevets

(11) EP 2 782 907 B1

(12) **EUROPEAN PATENT SPECIFICATION**

(45) Date of publication and mention of the grant of the patent:
**11.04.2018 Bulletin 2018/15**

(21) Application number: **12788603.4**

(22) Date of filing: **23.11.2012**

(51) Int Cl.:
***C07D 323/06*** *(2006.01)* ***C08G 2/36*** *(2006.01)*

(86) International application number:
**PCT/EP2012/073542**

(87) International publication number:
**WO 2013/076289 (30.05.2013 Gazette 2013/22)**

(54) **PROCESS FOR RECYCLING A FORMALDEHYDE SOURCE DURING A POLYMERIZATION PROCESS**

VERFAHREN ZUM RECYCLEN EINER FORMALDEHYDQUELLE WÄHREND EINES POLYMERISATIONSVORGANGES

PROCEDE DE RECYCLAGE D'UNE SOURCE DE FORMALDEHYDE AU COURS D'UN PROCESSUS DE POLYMERISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.11.2011 EP 11190567**
**24.11.2011 EP 11190574**
**24.11.2011 EP 11190586**
**25.10.2012 US 201261718393 P**
**25.10.2012 US 201261718557 P**

(43) Date of publication of application:
**01.10.2014 Bulletin 2014/40**

(73) Proprietor: **Celanese Sales Germany GmbH**
**65843 Sulzbach (DE)**

(72) Inventors:
- **KURZ, Klaus**
  **65451 Kelsterbach (DE)**
- **LINGNAU, Jürgen**
  **55130 Mainz-Laubenheim (DE)**
- **HAUBS, Michael**
  **55545 Bad Kreuznach (DE)**
- **HOFFMOCKEL, Michael**
  **65527 Niedernhausen (DE)**
- **FEORD, Damian**
  **F-67000 Strasbourg (FR)**

(74) Representative: **Lahrtz, Fritz et al**
**Isenbruck Bösl Hörschler LLP**
**Patentanwälte**
**Prinzregentenstrasse 68**
**81675 München (DE)**

(56) References cited:
**WO-A1-2007/023187** **WO-A1-2008/012232**

- **YAMAGUCHI T ET AL: "Synthesis of cyclooligomers of formaldehyde in liquid sulfur dioxide", CHEMISTRY & INDUSTRY, vol. 43, 23 October 1971 (1971-10-23), pages 1226-1227, XP008149518, SOCIETY OF CHEMICAL INDUSTRY, LONDON; GB ISSN: 0009-3068**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## BACKGROUND

Oxymethylene polymers, which include polyoxymethylene homopolymers and polyoxymethylene copolymers, possess many useful properties and characteristics. For example, the polymers can have great strength properties while also being chemical resistant. The polymers can also be easily molded into any desired shape. The polymers are currently being used in all different types of applications. For instance, polyoxymethylene polymers are being used to form interior or exterior automotive parts, parts for consumer appliances, parts for industrial processes, and the like.

Oxymethylene polymers can be produced via anionic polymerization of anhydrous formaldehyde or can be produced through the cationic polymerization of formaldehyde or cyclic oligomers, such as trioxane. During cationic polymerization, the polymer can be formed in bulk (i.e. without solvent). Alternatively, the polymerization can take place in solution where the polymer precipitates in a solvent to form a heterogeneous phase. In still another embodiment, a majority of the polymer may be formed in the heterogeneous phase followed by further polymerization in a homogeneous phase.

During the formation of oxymethylene polymers, cationic initiators are typically combined with one or more monomers to initiate polymerization. After polymerization, the reaction mixture can be rapidly and completely deactivated by adding a deactivator.

The deactivator can be added to a heterogeneous phase after the polymer has precipitated in a solvent, or can occur during a homogeneous phase, while the polymer is in a melted form. After being deactivated, the resultant polymer can be ground and/or pelletized. During the process, residual monomers may be drawn off. In the past, those skilled in the art have suggested reusing the residual monomers as starting materials for the polymer. For example, the residual monomers may be recirculated back to the polymerization reactor.

In the past, however, the residual monomers typically contained significant amounts of formaldehyde. Thus, the residual monomer was typically fed to a scrubbing column to remove formaldehyde, solvent, and/or water.

In view of the above, a need exists for a more efficient process for recovering residual monomers and formaldehyde during polymerization of oxymethylene polymers. A need also exists for a process for recovering formaldehyde and/or a formaldehyde source from the polymer process and for reusing the formaldehyde in an efficient manner. In addition, a need exists for a process for producing monomers for forming oxymethylene polymers that has a relatively high conversion rate.

## SUMMARY

The present invention is directed to a process for producing oxymethylene homo- or copolymers comprising: a) polymerizing at least one monomer to form an oxymethylene polymer in the presence of an initiator; b) deactivating the polymerization; c) removing residual monomers containing formaldehyde; d) contacting the formaldehyde with an aprotic compound having a boiling point of 120 °C or higher determined at 1 bar, and a catalyst; and e) at least partly converting the formaldehyde to a cyclic acetal. Furthermore, the present invention is directed to an oxymethylene homo- or copolymer obtainable according to the present invention. In general, the present disclosure is directed to a process for recovering formaldehyde and/or residual monomers during a process for producing oxymethylene polymers. In one embodiment, the recovered formaldehyde is converted into a cyclic acetal, such as trioxane. The cyclic acetal may then be used as a monomer during the process for producing the oxymethylene polymers. Any residual monomers collected with the formaldehyde can also be fed back into the polymer process.

In one embodiment, the process of the present disclosure for producing oxymethylene homo- or copolymers comprises the steps of at least partly polymerizing at least one monomer to form an oxymethylene polymer in the presence of an initiator. The at least one monomer may comprise a cyclic acetal in combination with at least one comonomer such as a cyclic ether, and optionally a regulator, such as methylal.

After a substantial portion of the monomers have been polymerized to form the oxymethylene polymer, the polymerization is deactivated by adding a deactivator. Prior to or after adding the deactivator, residual monomers containing formaldehyde are removed. In accordance with the present disclosure, the removed formaldehyde is contacted with an aprotic compound and a catalyst which at least partly converts the formaldehyde to a cyclic acetal. Of particular advantage, the formaldehyde can be contacted with the aprotic compound and the catalyst in the presence of any residual monomers. The cyclic acetal produced from the formaldehyde can, in one embodiment, be fed back to the process for producing the oxymethylene polymer.

The aprotic compound that is contacted with the formaldehyde may be in liquid form and may form a homogeneous phase with the formaldehyde as the formaldehyde is converted to the cyclic acetal in the presence of the catalyst. In one embodiment, for instance, the formaldehyde may comprise gaseous formaldehyde that is absorbed by the aprotic compound for contact with the catalyst.

The aprotic compound may be polar. For instance, in one embodiment, the aprotic compound may be dipolar. In one embodiment, the aprotic compound comprises a sulfur containing organic compound such as a sulfoxide, a sulfone, a sulfonate ester, or mixtures thereof. In one embodiment, the aprotic compound comprises sulfolane.

The aprotic compound may also have a relatively high static permittivity or dielectric constant of greater than about 15. The aprotic compound may also be nitro-group free. In particular, compounds having nitro-groups may form undesired side reactions within the process.

Once the cyclic acetal is formed from the formaldehyde, the cyclic acetal can be easily separated from the aprotic compound and the catalyst and fed back to the process for producing the oxymethylene polymer. In one embodiment, for instance, the cyclic acetal may be separated by distillation from the aprotic compound which may have a much higher boiling point than the cyclic acetal. The aprotic compound, for instance, may have a boiling point of greater than 120°C, such as greater than 140°C, such as greater than 160°C, such as even greater than 180°C at a pressure of one bar.

In one embodiment, the formaldehyde, the aprotic compound and the catalyst may form a reaction mixture that is primarily comprised of the aprotic compound. As described above, the aprotic compound may be in liquid form when contacted with the formaldehyde. When contacted with the aprotic compound, the formaldehyde may be in gaseous form or may be dissolved in a liquid, such as water to form an aqueous formaldehyde solution. The catalyst may form a homogeneous phase with the aprotic compound or may form a heterogeneous phase with the aprotic compound. For instance, the catalyst may comprise a solid.

In another embodiment, the present disclosure is directed to a process for recovering formaldehyde from an oxymethylene polymer production process. The process includes the steps of:

a) at least partly removing formaldehyde during or after the polymerization process,
b) contacting said formaldehyde with an aprotic compound and a catalyst; and
c) at least partly converting the formaldehyde to a cyclic acetal.

In one embodiment, the formaldehyde is removed as a gaseous mixture together with residual monomers. The gaseous mixture can then directly contact the aprotic compound and the catalyst without any intervening steps. Alternatively, the gaseous mixture may be fed through one or more different processes for removing selected components. For instance, in one embodiment, the gaseous mixture may be fed to a scrubber prior to being contacted with the aprotic compound and the catalyst.

Other features and aspects of the present disclosure are discussed in greater detail below.

## BRIEF DESCRIPTION OF THE DRAWINGS

A full and enabling disclosure of the present disclosure, including the best mode thereof to one of ordinary skill in the art, is set forth more particularly in the remainder of the specification, including reference to the accompanying figures in which:

FIG. 1 is a schematic diagram of one embodiment of a process in accordance with the present disclosure.

Repeat use of reference characters in the present specification and drawings is intended to represent same or analogous features or elements of the present disclosure.

## DETAILED DESCRIPTION

In general, the present disclosure is directed to a process for recovering byproducts from an oxymethylene polymer production process. The byproducts contain formaldehyde which, according to the present disclosure, is then converted into a cyclic acetal. The cyclic acetal may then be fed to the oxymethylene polymer production process for use as a monomer. Any residual monomers contained in the byproduct stream may also be fed back to the oxymethylene polymer production process.

The present disclosure is also directed to a process for producing a cyclic acetal. Of particular advantage, cyclic acetals can be produced from a formaldehyde source. As used herein, a formaldehyde source includes formaldehyde and oligomers or polymers formed from formaldehyde. Thus, a formaldehyde source can include paraformaldehyde, oxymethylene homopolymers, and oxymethylene copolymers.

In accordance with the present disclosure, the formaldehyde source is contacted with a catalyst in the presence of an aprotic compound to form a cyclic acetal. The aprotic compound facilitates production of the cyclic acetal in a manner that greatly enhances the conversion rates. Of particular advantage, the cyclic acetal produced according to the process can then be easily separated from the aprotic compound and the catalyst. For instance, in one embodiment, the cyclic acetal can be separated or isolated from the aprotic compound through a simple distillation process, since the

aprotic compound may have a much higher boiling point than the cyclic acetal.

In one embodiment, the aprotic compound is a liquid when contacted with the formaldehyde source. The formaldehyde source, on the other hand, may comprise gaseous formaldehyde, a liquid, or a solid. The formaldehyde source may dissolve into the aprotic compound or may be absorbed by the aprotic compound to form a homogeneous phase. The aprotic compound and the catalyst, in one embodiment, may comprise a liquid reaction mixture or a liquid medium.

The formaldehyde source reacts (converts) in the presence of a catalyst. Usually, cationic catalysts, such as Bronsted acids or Lewis acids, accelerate the conversion of the formaldehyde source to the desired cyclic acetals.

The catalyst is a catalyst for the conversion (reaction) of a formaldehyde source into cyclic acetals, in particular into trioxane and/or tetroxane.

Cyclic acetals within the meaning of the present disclosure relate to cyclic acetals derived from formaldehyde. Typical representatives are represented the following formula:

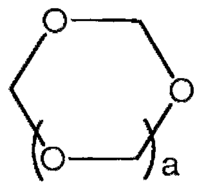

wherein a is an integer ranging from 1 to 3.

Preferably, the cyclic acetals produced by the process of the present disclosure are trioxane (a=1) and/or tetroxane (a=2). Trioxane and tetroxane usually form the major part (at least 80 wt.-%, preferably at least 90 wt.-%) of the cyclic acetals formed by the process of the present disclosure.

The weight ratio of trioxane to tetroxane varies with the catalyst used. Typically, the weight ratio of trioxane to tetroxane ranges from 3:1 to 40:1, preferably 4:1 to 20:1.

As described above, in one embodiment, the formaldehyde source that is used to produce the cyclic acetal is removed from an oxymethylene polymer process. The formaldehyde source, for instance, may comprise a monomer or byproduct produced during the polymer process. In accordance with the present disclosure, the formaldehyde source can be removed from the process, converted into a cyclic acetal as described above, and then fed back into the polymer process for producing the oxymethylene polymer.

The oxymethylene polymer production process may comprise any suitable process for producing oxymethylene homopolymers and/or copolymers. The polymer production process, for instance, may comprise an anionic polymerization process or a cationic polymerization process. The process for producing the oxymethylene polymer may comprise a heterogeneous process where the polymer precipitates in a liquid, may comprise a homogeneous process such as a bulk polymerization process that forms a molten polymer or may be a polymer process that includes both a heterogeneous phase and a homogeneous phase.

For the preparation of oxymethylene polymers, a monomer that forms - $CH_2$-O- units or a mixture of different monomers, are reacted in the presence of an initiator. Examples of monomers that form -$CH_2$O-units are formaldehyde or its cyclic oligomers, such as 1,3,5-trioxane(trioxane) or 1,3,5,7-tetraoxocane.

The oxymethylene polymers are generally unbranched linear polymers which generally contain at least 80 mol %, preferably at least 90 mol %, in particular at least 95 mol %, of oxymethylene units (-$CH_2$-O-). Alongside these, the oxymethylene polymers contain -($CH_2$)x-O- units, where x can assume the values from 2 to 25. Small amounts of branching agents can be used if desired. Examples of branching agents used are alcohols whose functionality is three or higher, or their derivatives, preferably tri- to hexahydric alcohols or their derivatives. Preferred derivatives are formulas in which, respectively, two OH groups have been reacted with formaldehyde, other branching agents include monofunctional and/or polyfunctional glycidyl compounds, such as glycidyl ethers. The amount of branching agents is usually not more than 1% by weight, based on the total amount of monomer used for the preparation of the oxymethylene polymers, preferably not more than 0.3% by weight.

Oxymethylene polymers can also contain hydroxyalkylene end groups - O-($CH_2$)x-O alongside methoxy end groups, where x can assume the values from 2 to 25. These polymers can be prepared by carrying out the polymerization in the presence of diols of the general formula HO-$(CH_2)_x$-OH, where x can assume the values from 2 to 25. The polymerization in the presence of the diols leads, via chain transfer, to polymers having hydroxyalkylene end groups. The concentration of the diols in the reaction mixture depends on the percentage of the end groups intended to be present in the form of -O-$(CH_2)_x$-OH, and is from 10 ppm by weight to 2 percent by weight.

The molecular weights of these polymers, expressed via the volume melt index MVR, can be adjusted within a wide range. The polymers typically have repeat structural units of the formula -$(CH_2\text{-}O\text{-})_n$-, where n indicates the average degree of polymerization (number average) and preferably varies in the range from 100 to 10 000, in particular

from 500 to 4000.

Oxymethylene polymers can be prepared in which at least 80%, preferably at least 90%, particularly preferably at least 95%, of all of the end groups are alkyl ether groups, in particular methoxy or ethoxy groups.

Comonomers that may be used to produce oxymethylene copolymers including cyclic ethers or cyclic formals. Examples include, for instance, 1,3-dioxolane, diethylene glycol formal, 1,4-butanediol formal, ethylene oxide, propylene 1,2-oxide, butylene 1,2-oxide, butylene 1,3-oxide, 1,3 dioxane, 1,3,6-trioxocane, and the like. In general, one or more of the above comonomers may be present in an amount from 0.1 to 20 mol %, such as from 0.2 to 10 mol %, based on the amount of trioxane.

The molecular weight of the resultant homo- and copolymers can be adjusted via use of acetals of formaldehyde (chain transfer agents). These also lead to production of etherified end groups of the polymers, and a separate reaction with capping reagents can therefore be omitted. Chain transfer agents used are monomeric or oligomeric acetals of formaldehyde. Preferred chain transfer agents are compounds of the formula I

$$R^1\text{-}(O\text{-}CH_2)_q\text{-}O\text{-}R^2 \qquad (I),$$

in which $R^1$ and $R^2$, independently of one another, are monovalent organic radicals, preferably alkyl radicals, such as butyl, propyl, ethyl, and in particular methyl, and q is a whole number from 1 to 50.

Particularly preferred chain transfer agents are compounds of the formula I, in which q=1, very particularly preferably methylal.

The amounts used of the chain transfer agents are usually up to 5000 ppm, preferably from 100 to 3000 ppm, based on the monomer (mixture).

The initiators used can comprise the cationic initiators usually used in the preparation of oxymethylene homo- and copolymers. Examples of these are protic acids, e.g. fluorinated or chlorinated alkyl- and arylsulfonic acids, such as trifluoromethanesulfonic acid, trifluoromethanesulfonic anhydride, or Lewis acids, such as stannic tetrachloride, arsenic pentafluoride, phosphorus pentafluoride, and boron trifluoride, and also their complex compounds, e.g. boron trifluoride etherate, and carbocation sources, such as triphenylmethyl hexafluorophosphate.

In one embodiment, the initiator for cationic polymerization is an isopoly acid or a heteropolyacid or an acid salt thereof which may be dissolved in an alkyl ester of a polybasic carboxylic acid.

The heteropoly acid is a generic term for polyacids formed by the condensation of different kinds of oxo acids through dehydration and contains a mono- or poly-nuclear complex ion wherein a hetero element is present in the center and the oxo acid residues are condensed through oxygen atoms. Such a heteropoly acid is represented by formula (1):

$$H_x[M_mM'_nO_z] \blacksquare yH_2O \qquad (1)$$

wherein

M represents an element selected from the group consisting of P, Si, Ge, Sn, As, Sb, U, Mn, Re, Cu, Ni, Ti, Co, Fe, Cr, Th and Ce,

M' represents an element selected from the group consisting of W, Mo, V and Nb, m is 1 to 10,

n is 6 to 40,

z is 10 to 100,

x is an integer of 1 or above, and

y is 0 to 50.

According to a preferred embodiment of the method according to the present invention the heteropoly acid is a compound represented by the following formula:

$$H_x[M_mM'_nO_z] \blacksquare yH_2O$$

wherein

M represents an element selected from the group consisting of P and Si;

M' represents a coordinating element selected from the group consisting of W, Mo and V;

z is 10 to 100;

m is 1 to 10;

n is 6 to 40;

x is an integer of at least 1; and

y is 0 to 50.

The central element (M) in the formula described above may be composed of one or more kinds of elements selected from P and Si and the coordinate element (M') may be composed of at least one element selected from W Mo and V, particularly preferably W or Mo.

Further, acidic salts of heteropoly acids each having a form, in which any of the various metals substitutes for a part of H's (hydrogen atoms) in the formula (1) can also be used as the initiator.

Specific examples of heteropoly acids are selected from the group consisting of phosphomolybdic acid, phosphotungstic acid, phosphomolybdotungstic acid, phosphomolybdovanadic acid, phosphomolybdotungstovanadic acid, phosphotungstovanadic acid, silicotu ngstic acid, silicomolybdic acid, silicomolybdotungstic acid, silicomolybdotungstovanadic acid and acid salts thereof.

Excellent results have been achieved with heteropoly acids selected from 12- molybdophosphoric acid ($H_3PMO_{12}O_{40}$) and 12-tungstophosphoric acid ($H_3PW_{12}O_{40}$) and mixtures thereof.

The amount of the heteropoly acid or the acid salt thereof to be used as a initiator for the polymerization of a monomer component, which forms - $CH_2$-O-units is 0.1 to 1000 ppm, preferably 0.2 to 40 ppm, more preferably 0.3 to 5 ppm based on the total amount of the monomer component.

In another embodiment, the initiator for cationic polymerization comprises at least one protic acid and at least one salt of a protic acid, wherein said at least one protic acid is sulfuric acid, tetrafluoroboric acid, perchloric acid, fluorinated alkyl sulfonic acid, chlorinated alkyl sulfonic acid or aryl sulfonic acid, and wherein said salt of protic acid is an alkali metal or alkaline earth metal salt of protic acid and/or a substituted ammonium salt of protic acid, the cations of the ammonium salt having the general formula (I)

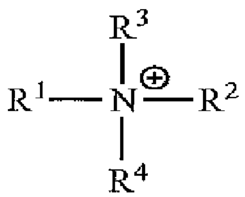

(I)

where $R^1$-$R^4$ are independently hydrogen, an alkyl group or an aryl group.

Particular preference is given to substituted ammonium ions having the general formula (I)

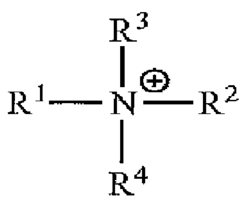

(I)

where $R^1$ to $R^4$ are independently hydrogen, an alkyl group such as methyl, ethyl, n-propyl, isopropyl, n-butyl, isobutyl or an aryl group such as phenyl or 4-methoxypheny.

Substituted ammonium ions are also preferred because the corresponding salts are very simple to prepare by mixing the protic acid with the corresponding amine. Thus, mixing triethylamine and trifluoromethanesulfonic acid forms triethylammonium triflate.

Useful organic cations further include protonated nitrogenous compounds, examples being protonated imidazole and protonated amides. Useful amides include for example dimethylformamide, dimethylacetamide and N-methylpyrrolidone.

The anions of the salts are chosen for low nucleophilicity and good thermal stability. Examples are perchlorate, tetrafluoroborate, tetraphenylborate, hexafluorophosphate and the preferred trifluorometha nesulfonate.

The molar ratio of protic acid to salt can be varied within a wide window. In principle, molar ratios of protic acid to salt in the range from 1:0.01 to 1:2000 are possible, preferably in the range from 1:0.5 to 1:10, more preferably in the range from 1:0.8 to 1:8 and most preferably in the range from 1:1 to 1:4.

The amount of the above initiator used is in the range from $10^{-6}$% by weight to 1 % by weight, preferably in the range from $10^{-5}$% by weight to $10^{-3}$% by weight and more preferably in the range from 2x $10^{-5}$% by weight to 5x $10^{-4}$% by weight, based on the total weight of monomers used. The amount of initiator used depends on the chemical composition of the protic acid and the chemical composition of the monomers or monomer mixture. For example, typically less initiator is used for homopolymerizing 1,3,5-trioxane than for copolymerizing trioxane with dioxolane.

In order to terminate the polymerization, the reaction mixture, which still comprises unconverted monomers and/or byproducts, such as trioxane and formaldehyde, alongside polymer, is brought into contact with deactivators. These can be added in bulk form or a form diluted with an inert solvent to the polymerization mixture. The result is rapid and complete deactivation of the active chain ends.

Deactivators that can be used are those compounds which react with the active chain ends in such a way as to terminate the polymerization reaction. Examples are the organic bases triethylamine or melamine, and also the inorganic bases potassium carbonate or sodium acetate. It is also possible to use very weak organic bases, such as carboxamides, e.g. dimethylformamide. Tertiary bases are particularly preferred, examples being triethylamine and hexamethylmelamine.

The concentrations used of the bases are preferably from 1 ppm to 1 % by weight, based on the polymerization material. Concentrations of from 10 ppm to 5000 ppm are preferred.

The polymerization can occur in any suitable reactor. Referring to FIG. 1, merely for exemplary purposes, one embodiment of a process for producing oxymethylene polymers is illustrated. The process illustrated in FIG. 1 is a continuous process in which at least one monomer is continuously polymerized to form the oxymethylene polymer. During the process, formaldehyde and any other residual monomers are withdrawn and fed to an apparatus for converting the formaldehyde to a cyclic acetal in accordance with the present disclosure.

As shown in FIG. 1, the process includes a polymerization reactor 10. One or more monomers are fed to the polymerization reactor 10 in combination with an initiator. For instance, the monomers may comprise trioxane alone or in combination with a comonomer, such as 1,3 dioxolane. In the embodiment illustrated in FIG. 1, polymerization initially takes place in the heterogeneous phase which means that a solid polymer precipitates alongside monomer which has not yet been consumed. If desired, a chain transfer agent, such as acetals or formaldehyde, or a regulator, such as methylal, may also be added to the reactor. Heterogeneous polymerization can be carried out at a temperature ranging from 60°C to 150°C, such as from 80°C to 140°C. The pressure is typically from 15 bar to 100 bar, such as from 25 bar to 50 bar.

During initial polymerization, the temperature and pressure are set such that the polymer substantially precipitates in the reaction mixture forming a solid/liquid mixture. The polymerization conversion is from 10% to 80%, such as from 50% to 70%.

In one embodiment, once polymerization is initiated in the heterogeneous phase, the temperature within the reactor 10 rises such that the solid/liquid mixture becomes substantially homogeneous. The rise in temperature can occur in a single reactor as shown in FIG. 1 such that a continuous transition is present in the reactor between the heterogeneous phase and the substantially homogeneous phase. In an alternative embodiment, however, the heterogeneous phase can take place in a first reactor, while the homogeneous phase can take place in a second and separate reactor.

The temperature rise that causes formation of a substantially homogeneous phase can be brought about by applying heat to a portion of the reactor 10. In one embodiment, the heat of polymerization/crystallization can also be used to raise the temperature. Ultimately, the temperature and pressure within the reactor 10 can be controlled such that polymerization is carried out with a certain temperature profile. A controlled temperature profile permits adjustment as desired of some of the properties of the polymer. The controlled utilization of the heat of polymerization/crystallization permits efficient utilization of energy.

The temperature profile over the entire polymerization typically varies from 65°C initially to 250°C prior to deactivation. The temperature and residence time during the homogeneous phase, in one embodiment, may be minimized in order to suppress undesired side reactions. The homogeneous phase can be carried out at a temperature from 150°C to 250°C, such as from 160°C to 200°C.

After a desired amount of time, the homogeneous phase is deactivated by adding a deactivator to the polymerization reactor 10. In other embodiments, however, deactivation may occur prior to substantially reaching the homogeneous phase.

As shown in FIG. 1, in one embodiment, the polymer melt is fed from the polymerization reactor 10 to a hydrolysis chamber 20. Although hydrolysis is completely optional, hydrolysis can remove unstable end groups, such as formiate end groups and/or hemiacetal groups. Hydrolysis can lead to an unzipping of formaldehyde units from the chain ends up to the first hydrolysis stable group. Consequently, hydrolysis produces a more stable oxymethylene polymer.

Hydrolysis in the chamber 20 can occur using various techniques and processes. For example, in one embodiment, the deactivated oxymethylene polymer can be heated to an elevated temperature to remove the unstable end groups in a process known as thermalhydrolysis. During thermalhydrolysis, the polymerization mixture may be heated to a temperature greater than 160°C, such as a temperature greater than 170°C, such as a temperature of from 180° C to 220°C, such as from 180°C to 200°C. In one embodiment, the polymer may be heated to a temperature of 190°C for 20 minutes.

In an alternative embodiment, the hydrolysis chamber 20 may contain a mixture of one or more solvents. The solvents may comprise, for instance, water, methanol, ethanol, and/or isopropanol. In one embodiment, for instance, a hydrolysis chamber 20 may contain a hydrolysis mixture comprising water and methanol. The temperature of the hydrolysis mixture may be from 160°C to 220°C.

As shown in FIG. 1, the oxymethylene polymer may be conveyed from the hydrolysis chamber 20 to an extruder/devolatilizer 30. In extruder 30, the polymer may be extruded into pellets while simultaneously removing volatile components, which may comprise residual monomers including formaldehyde. The volatile components can be removed using various techniques. For instance, in one embodiment, the volatile components can be removed from the extruder 30 through reduced pressure aspiration. In particular, the volatile constituents can be drawn off by way of suction from the mixture being fed through the extruder 30. As shown in FIG. 1, the volatile components can be drawn off the extruder at multiple locations.

In an alternative embodiment, a gas can be fed through the extruder 30 that collects the volatile components. The gas, for instance, may be an inert gas such as nitrogen.

In accordance with the present disclosure, the volatile components collected from the polymerization process are then fed to a reactor 40 that is designed to convert any formaldehyde contained in the gas stream into one or more cyclic acetals. In the embodiment illustrated in FIG. 1, the volatile components are drawn off from the extruder 30. It should be understood, however, that the volatile components can be separated from the polymer process in any desired location. In general, the volatile components are collected after a substantial portion of the polymerization has occurred. For instance, the volatile components may be removed after at least 60%, such as at least 70%, such as at least 80% of the monomers fed to the process are converted into the oxymethylene polymer.

The volatile components collected during the polymer process contain formaldehyde, residual monomers, and possibly other volatile constituents. As shown in FIG. 1, the byproduct stream 35 containing the volatile components can be fed directly to the reactor 40. In other embodiments, however, various pretreatment steps may occur prior to feeding the volatile components to the reactor 40. For instance, in one embodiment, volatile components may be fed to a distillation column or scrubbing device for removing various unwanted constituents prior to feeding the volatile components to the reactor 40. The reactor 40 is well suited to receiving formaldehyde and residual monomers, such as trioxane.

In one embodiment, the volatile components may be fed to the reactor 40 in the form of a gas. For instance, the volatile components may contain gaseous formaldehyde which is fed to the reactor 40. In this embodiment, the reactor 40 may comprise a counter current scrubber. in particular, the aprotic compound optionally combined with a catalyst can flow downwards through the reactor 40 and contact the gaseous formaldehyde for conversion into a cyclic acetal. The catalyst may be a liquid that has been combined with the aprotic compound. In an alternative embodiment, the catalyst may comprise a solid contained within the reactor.

In an alternative embodiment, formaldehyde removed from the polymer process may be contained in an aqueous solution and fed to the reactor 40. In the reactor 40, the aqueous formaldehyde solution may be combined with the aprotic compound and a catalyst for conversion into a cyclic acetal.

As shown in FIG. 1, in one embodiment, any cyclic acetals produced in the reactor 40 can then be fed back into the polymer process for producing the oxymethylene polymer. In one embodiment, newly formed cyclic acetals may be combined with residual monomers removed from the polymer process and fed back to the polymerization reactor 10.

As described above, formaldehyde or a formaldehyde source removed from the oxymethylene process is converted to a cyclic acetal by contacting the formaldehyde source with aprotic compound and a catalyst. As used herein, an aprotic compound is a compound that does not contain any substantial amounts of hydrogen atoms which can disassociate.

In one embodiment, the aprotic compound is liquid under the reaction conditions. Therefore, the aprotic compound may have a melting point of 180°C or less, preferably 150°C or less, more preferably 120°C or less, especially 60°C or less.

For practical reasons, it is advantageous to use an aprotic compound which has a melting point in the order of

preference (the lower the melting point the more preferred) of below 50°C, below 40°C and below 30°C and below 20°C. Especially, aprotic compounds which are liquid at 25 or 30°C are suitable since they can be easily transported by pumps within the production plant.

Further, the aprotic compound has a boiling point of 120 °C or higher, determined at 1 bar. Preferably the aprotic compound may have a boiling point of 140 °C or higher, more preferably 160 °C or higher, especially 180 °C or higher, determined at 1 bar. In a further embodiment the boiling point of the aprotic compound is 200 °C or higher, preferably 230 °C or higher, more preferably 240 °C or higher, further preferably 250 °C or higher and especially 260 °C or higher or 270 °C or higher. The higher the boiling point the better the cyclic acetals, especially trioxane and/or tetroxane, formed by the process of the present disclosure can be separated by distillation. Therefore, according to an especially preferred embodiment of the present disclosure the boiling point of the aprotic compound is at least 20 °C higher than the boiling point of the cyclic acetal formed, in particular at least 20 °C higher than the boiling point of trioxane and/or tetroxane.

Additionally, aprotic compounds are preferred which do not form an azeotrope with the cyclic acetal, especially do not form an azeotrope with trioxane.

In a preferred embodiment of the present invention the reaction mixture or liquid medium in the reactor 40 comprises at least 20 wt.-%, preferably at least 40 wt.-%, more preferably at least 60 wt.-%, most preferably at least 80 wt.-% and especially at least 90 wt.-% of the aprotic compound(s), wherein the weight is based on the total weight of the reaction mixture. The liquid medium or the reaction mixture or the liquid mixture (A) may comprise one or more aprotic compound(s).

In a preferred embodiment the liquid medium is essentially consisting of the aprotic compound. Essentially consisting of means that the liquid medium comprises at least 95 wt.-%, preferably at least 98 wt.-%, more preferably at least 99 wt.-%, especially at least 99.5 wt.-%, in particular at least 99.9 wt.-% of the aprotic compound(s). In a further embodiment of the invention the liquid medium is the aprotic compound, i.e., the liquid medium is consisting of the aprotic compound.

It has been found that liquid aprotic compounds which at least partly dissolve or absorb the formaldehyde source lead to excellent results in terms of conversion of the formaldehyde source into the desired cyclic acetals.

Therefore, aprotic compounds are preferred which at least partly dissolve or absorb the formaldehyde source under the reaction conditions. Preferred are aprotic compounds which dissolve paraformaldehyde (98 wt.-% formaldehyde, 2 wt.-% water) [can also be expressed as Pn = moles of formaldehyde/moles of water = (98/30) / (2/18) = approx. 29] at the reaction temperature in an amount of at least 0.1 wt.-%, wherein the weight is based on the total weight of the solution.

The aprotic compound used in the process can be a polar aprotic compound, especially a dipolar compound. Polar aprotic solvents are much more suitable to dissolve the formaldehyde source. Non-polar aprotic compounds such as unsubstituted hydrocarbons (e.g. cyclic hydrocarbons such as cyclohexane, or alicyclic hydrocarbons such as hexane, octane, decane, etc.) or unsubstituted unsaturated hydrocarbons or unsubstituted aromatic compounds are less suitable. Therefore, according to a preferred embodiment the aprotic compound is not an unsubstituted hydrocarbon or unsubstituted unsaturated hydrocarbon or unsubstituted aromatic compound. Further, preferably the reaction mixture comprises unsubstituted hydrocarbons and/or unsubstituted unsaturated hydrocarbons and/or unsubstituted aromatic compounds in an amount of less than 50 wt.-%, more preferably less than 25 wt.-%, further preferably less than 10 wt.-%, especially less than 5 wt.-%, e.g. less than 1 wt.-% or 0 wt.-%.

Halogen containing compounds are less preferred due to environmental aspects and due to their limited capability to dissolve the formaldehyde sources. Further, the halogenated aliphatic compounds may cause corrosions in vessels or pipes of the plant and it is difficult to separate the cyclic acetals formed from the halogenated compounds.

According to one embodiment, the aprotic compound is halogen free. In a further preferred embodiment the reaction mixture comprises less than 50 wt.-%, more preferably less than 25 wt.-%, further preferably less than 10 wt.-%, more preferably less than 5 wt.-%, especially less than 1 wt.-% or 0 wt.-% of halogenated compounds.

Likewise, the use of (liquid) sulphur dioxide leads to difficulties with isolation of the cyclic acetals. Therefore, the aprotic compound is preferably free of sulphur dioxide. In a further preferred embodiment the reaction mixture comprises less than 50 wt.-%, more preferably less than 25 wt.-%, further preferably less than 10 wt.-%, more preferably less than 5 wt.-%, especially less than 1 wt.-% or 0 wt.-% of sulphur dioxide.

Polar aprotic compounds are especially preferred. According to a preferred embodiment of the invention the aprotic compound has a relative static permittivity of more than 15, preferably more than 16 or more than 17, further preferably more than 20, more preferably of more than 25, especially of more than 30, determined at 25 °C or in case the aprotic compound has a melting point higher than 25 °C the relative permittivity is determined at the melting point of the aprotic compound.

The relative static permittivity, $\varepsilon_r$, can be measured for static electric fields as follows: first the capacitance of a test capacitor $C_o$, is measured with vacuum between its plates. Then, using the same capacitor and distance between its plates the capacitance $C_x$ with an aprotic compound between the plates is measured. The relative dielectric constant

can be then calculated as

$$\varepsilon_r = \frac{C_x}{C_0}.$$

Within the meaning of the present invention the relative permittivity is determined at 25°C or or in case the aprotic compound has a melting point higher than 25°C the relative permittivity is determined at the melting point of the aprotic compound.

According to a further aspect of the invention the aprotic compound is a dipolar aprotic compound.

The aprotic compound within the meaning of the present invention is generally a dipolar and non-protogenic compound which has a relative permittivity as defined above of more than 15, preferably more than 25 or more than 30, determined at 25°C or in case the aprotic compound has a melting point higher than 25°C the relative permittivity is determined at the melting point of the aprotic compound.

The process can be carried out in manner wherein the formaldehyde source is completely dissolved or absorbed in the liquid medium or reaction mixture or liquid mixture (A).

Therefore, according to one embodiment the formaldehyde source and the aprotic compound form a homogenous phase under the reaction conditions.

Suitable aprotic compounds are selected from the group consisting of organic sulfoxides, organic sulfones, organic sulfonate ester, and mixtures thereof.

According to a preferred embodiment the aprotic compound is selected from sulfur containing organic compounds.

Further, the aprotic compound is preferably selected from the group consisting of cyclic or alicyclic organic sulfoxides, alicyclic or cyclic sulfones, and mixtures thereof.

Excellent results can be achieved by aprotic compounds as represented by the following formula (I):

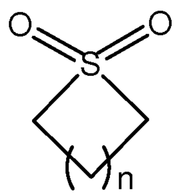

(I)

wherein

n is an integer ranging from 1 to 6, preferably 2 or 3, and

wherein the ring carbon atoms may optionally be substituted by one or more substituents, preferably selected from $C_1$-$C_8$-alkyl which may be branched or unbranched. Preferred compounds of formula (I) are sulfolane, methylsulfolane, dimethylsulfolane, ethylsulfolane, diethylsulfolane, propylsulfolane, dipropylsulfolane, butylsulfolane, dibutylsulfolane, pentylsulfolane, dipentylsulfolane, and hexylsulfolane as well as octylsulfolane.

According to the most preferred embodiment the aprotic compound is sulfolane (tetrahydrothiophene-1,1-dioxide).

Sulfolane is an excellent solvent for the formaldehyde source, it is stable under acidic conditions, it does not deactivate the catalysts and it does not form an azeotrope with trioxane. Further, it is a solvent which is inert under the reaction conditions.

Unless indicated otherwise the expression "reaction mixture" refers to the mixture which is used for the reaction of the formaldehyde source to the cyclic acetals. The concentrations and amounts of the individual components of the reaction mixture refer to the concentrations and amounts at the beginning of the reaction. In other words the reaction mixture is defined by the amounts of its starting materials, i.e. the amounts of initial components.

Likewise the amounts defined for the "liquid mixture (A)" refer to the amounts of the components at the beginning of the reaction, i.e. prior to the reaction.

The formaldehyde source reacts to the cyclic acetals and, as a consequence, the concentration of the formaldehyde source decreases while the concentration of the cyclic acetals increases.

At the beginning of the reaction a typical reaction mixture of the invention comprises a formaldehyde source

which is at least partly, preferably completely dissolved or absorbed in sulfolane and a catalyst.

Further, an especially preferred embodiment of the present invention is a process for producing cyclic acetal comprising reacting a formaldehyde source in the presence of a catalyst wherein the reaction is carried out in sulfolane or a process for producing cyclic acetal from a formaldehyde source in the presence of a catalyst and sulfolane.

A further preferred aprotic compound is represented by formula (II):

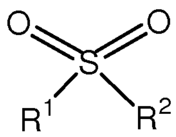

(II)

wherein $R^1$ and $R^2$ are independently selected from $C_1$-$C_8$-alkyl which may be branched or unbranched, preferably wherein $R^1$ and $R^2$ independently represent methyl or ethyl. Especially preferred is dimethyl sulfone.

According to a further preferred embodiment the aprotic compound is represented by formula (III):

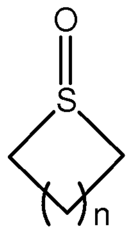

(III)

wherein

n is an integer ranging from 1 to 6, preferably 2 or 3, and

wherein the ring carbon atoms may optionally be substituted by one or more substituents, preferably selected from $C_1$-$C_8$-alkyl which may be branched or unbranched.

Suitable aprotic compounds are also represented by formula (IV):

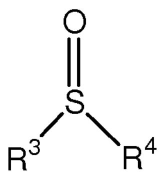

(IV)

wherein $R^3$ and $R^4$ are independently selected from $C_1$-$C_8$-alkyl which may be branched or unbranched, preferably wherein $R^1$ and $R^2$ independently represent methyl or ethyl.

Especially preferred is dimethyl sulfoxide.

Suitable aprotic compounds may be selected from aliphatic dinitriles, preferably adiponitrile.

In a further aspect of the invention a mixture of two or more aprotic compounds is used. A mixture of aprotic compounds may be used to decrease the melting point of the aprotic medium. In a preferred embodiment the aprotic compound comprises or is consisting of a mixture of sulfolane and dimethyl sulfoxide.

The process of the invention is carried out in the presence of a catalyst for the conversion of the formaldehyde source into cyclic acetals. Suitable catalysts are any components which accelerate the conversion of the formaldehyde source to the cyclic acetals.

The catalyst is a catalyst for the conversion (reaction) of a formaldehyde source into cyclic acetals, preferably into trioxane and/or tetroxane.

Usually, cationic catalysts can be used for the process of the invention. The formation of cyclic acetals can be heterogeneously or homogenously catalysed. In case the catalysis is heterogeneous the liquid mixture comprising the formaldehyde source and the aprotic compound is contacted with the solid catalyst or an immiscible liquid catalyst. A typical liquid immiscible catalyst is a liquid acidic ion exchange resin. Solid catalyst means that the catalyst is at least partly, preferably completely in solid form under the reaction conditions. Typical solid catalysts which may be used for

the process of the present invention are acid ionexchange material, Lewis acids and/or Bronsted acids fixed on a solid support, wherein the support may be an inorganic material such as $SiO_2$ or organic material such as organic polymers.

However, preferred is a homogenous catalysis wherein the catalyst is dissolved in the reaction mixture.

Preferred catalysts are selected from the group consisting of Bronsted acids and Lewis acids. The catalyst is preferably selected from the group consisting of trifluoromethanesulfonic acid, perchloric acid, methanesulfonic acid, toluenesulfonic acid and sulfuric acid, or derivatives thereof such as anhydrides or esters or any other derivatives that generate the corresponding acid under the reaction conditions. Lewis acids like boron trifluoride, arsenic pentafluoride can also be used. It is also possible to use mixtures of all the individual catalysts mentioned above.

The catalyst is typically used in an amount ranging from 0.001 to 15 wt%, preferably 0.01 to 5 wt% or 0.01 to 10 wt.-%, more preferably from 0.05 to 2 wt% and most preferably from 0.05 to 0.5 wt%, based on the total weight of the reaction mixture.

Advantageously, the aprotic compound does not essentially deactivate the catalyst. Generally, the catalysts used for the formation of cyclic acetals from a formaldehyde source are cationic catalysts, such as Bronsted acids or Lewis acids. Preferably, under the reaction conditions the aprotic compound does essentially not deactivate the catalyst used in the process of the present invention. Aprotic solvents such as dimethylformamide (DMF), dimethylacetamide (DMAC) or N-methylpyrrolidone (NMP) are too basic and therefore may deactivate the catalyst and, as a consequence, said solvents are less suitable. According to a preferred embodiment of the present invention the liquid reaction mixture is essentially free of amides, preferably essentially free of acylic or cyclic amides. Essentially free means that the amides may be present in an amount of less than 5 wt.-%, preferably less than 2 wt.-%, more preferably less than 0.5 wt.-%, especially less than 0.01 wt.-% and, in particular, less than 0.001 wt.-% or 0 wt.-%, wherein the weight is based on the total weight of the liquid reaction mixture.

Nitro group containing compounds can lead to undesired side products or even demonstrate an insufficient solubility for the formaldehyde sources.

Therefore, the aprotic compound preferably does not comprise a nitro group and/or a nitrogen atom. Further, according to a preferred embodiment of the present invention the aprotic compound is a non-aromatic aprotic compound. Especially, the aprotic compound is not nitrobenzene or an aromatic nitro compound. Further, preferably, the aprotic compound does not comprise ether.

Within the meaning of the present invention the aprotic compound does not deactivate the catalyst if under the reaction conditions less than 95 %, preferably less than 50 %, more preferably less than 10 %, of the Bronsted acid catalyst used protonates the aprotic compound. In case a Lewis acid catalyst is used the aprotic compound does not deactivate the catalyst if under the reaction conditions less than 90 wt-%, preferably less than 50 wt.-%, more preferably less than 10 wt-% of the Lewis acid catalyst forms a complex with the aprotic compound.

The degree of protonation and complex formation can be determined by NMR spectroscopy such as $^1H$ or $^{13}C$-NMR. The degree of protonation and complex formation is determined at 250°C, preferably in $d_6$-DMSO.

The deactivation of the catalyst can also be determined in the following manner:

10 g of commercially available paraformaldehyde (95 wt%) is dissolved in 100 g of sulfolane at a temperature sufficient to dissolve the paraformaldehyde in such a way that no gaseous formaldehyde can escape. The clear solution is kept at 90° C and 0.1 wt% of triflic acid is added. The rate of the formation of trioxane is measured (by measuring the concentration of trioxane as a function of time).

The same experiment is repeated, except that 10 g of the sulfolane are replaced by 10 g of the aprotic compound to be tested. If the rate of trioxane formation is still greater than 1 %, preferably greater than 5 %, more preferably greater than 10 %, of the rate of the initial experiment then it is concluded that the aprotic compound in question does not deactivate the catalyst (even though it may reduce its activity).

The aprotic compound should not be too basic in order to avoid deactivation of the catalysts. On the other hand the aprotic compound preferably does not chemically react with the formaldehyde source under the reaction conditions, i.e. is an inert aprotic compound.

Preferably, under the reaction conditions the aprotic compound should not react chemically with the formaldehyde source or the cyclic acetal obtained by the process of the invention. Compounds like water and alcohols are not suitable as they react with formaldehyde. Within the meaning of the present invention an aprotic compound does not chemically react with the formaldehyde source when it meets the following test criteria:

5 g of commercially available paraformaldehyde (95 wt.-%) is added to 100 g of the aprotic compound containing 0.1 wt.-% trifluoromethanesulfonic acid and heated at 120°C for 1 hour with stirring in a closed vessel so that no gaseous formaldehyde can escape. If less than 1 wt.-%, preferably less than 0.5 wt.-%, more preferably less than 0.1 wt.-% and most preferably less than 0.01 wt.-% of the aprotic compound has chemically reacted, then the aprotic compound is considered not to have reacted with the formaldehyde source. If the aprotic compound meets the criteria it is considered inert.

Further, under the acidic reaction conditions the aprotic compound should be essentially stable. Therefore, aliphatic ethers or acetals are less suitable as aprotic compounds. The aprotic compound is considered stable under

acidic conditions within the meaning of the present invention if the aprotic compound meets the following test conditions:

100 g of the aprotic compound to be tested containing 0.5 % by weight (wt.-%) trifluoromethanesulfonic acid is heated at 120°C for 1 hour. If less than 0.5 wt.-%, preferably less than 0.05 wt.-%, more preferably less than 0.01 wt.-% and most preferably less than 0.001 wt.-% of the aprotic compound has chemically reacted, then the aprotic compound is considered to be stable under acidic conditions.

The formaldehyde source used in the process and reaction mixture and liquid mixture (A) of the present invention can in principle be any compound which can generate formaldehyde or which is formaldehyde or an oligomer or (co) - polymer thereof. In the process of the present disclosure, the formaldehyde source is generally formaldehyde that comprises gaseous formaldehyde or comprises an aqueous solution of formaldehyde.

In general, better conversion efficiencies are achieved when gaseous formaldehyde is fed to the reactor 40 as shown on FIG. 1. In particular, conversion and yield can be improved when the amount of water contained in the reactor 40 is minimized.

According to a further aspect the water content and/or the content of protic compounds of the formaldehyde source is less than 10000 ppm, preferably less than 1000 ppm, most preferably less than 100 ppm, such as 5 to 80 ppm, wherein the ppm (parts per million) refer to the total weight of the formaldehyde source mixture.

When contained in an aqueous solution, the formaldehyde content of the aqueous formaldehyde solution is preferably ranging from 60 to 90 wt.-%, more preferably ranging from 65 to 85 wt.-%, based on the total weight of the aqueous formaldehyde solution.

The process of the invention can also be used to change the ratio of cyclic acetals derived from formaldehyde. Therefore, the formaldehyde source can also comprise cyclic acetals selected from the group consisting of trioxane, tetroxane and cyclic oligomers derived from formaldehyde.

Preferably, the reaction mixture comprises the formaldehyde source in an amount ranging from 0.1 to 80 wt% or 1 to less than 80 wt.-%, more preferably from 5 to 75 wt%, further preferably ranging from 10 to 70 wt% and most preferred ranging from 20 to 70 wt%, especially ranging from 30 to 60 wt.-% based on the total weight of the reaction mixture.

It has been found that especially good results in terms of conversion can be achieved when the formaldehyde source is dissolved in a high concentration in the aprotic compound.

Therefore, in a further aspect the amount of formaldehyde source is at least 5 wt.-% or at least 10 wt.-%, preferably ranging from 5 to 75 wt.-%, further preferably 10 to 70 wt.-%, especially 15 to 60 wt.-%, based on the total weight of the homogeneous liquid mixture consisting of the formaldehyde source and the aprotic compound.

According to a preferred embodiment the weight ratio of formaldehyde source to aprotic compound is ranging from 1:1000 to 4:1, preferably 1:600 to 3:1, more preferably 1:400 to 2:1, further preferably 1:200 to 1:1, especially preferably 1:100 to 1:2, particularly 1:50 to 1:3, for example 1:20 to 1:6 or 1:15 to 1:8.

Typically, the reaction is carried out at a temperature higher than about 0°C, preferably ranging from 0°C to 150°C, more preferably ranging from 10°C to 120°C, further preferably from 20°C to 100 °C and most preferably from 30°C to 90°C.

In a further aspect of the invention the reaction can be carried out at a temperature higher than 0°C, preferably ranging from 0°C to 200°C, more preferably ranging from 20°C to 150°C, further preferably ranging from 40°C to 130°C and most preferably from 60°C to 120°C, especially from 80°C to 120°C or from 80°C to 100°C.

The pressure during the reaction can generally be from 10 millibars to 20 bars, such as from 0.5 bar to 10 bar, such as from 0.5 bar to 2 bar.

A further advantageous of the process of the present invention is that the cyclic acetals can easily be separated from the reaction mixture. The cyclic acetal, especially the trioxane can be separated from the reaction mixture by distillation in a high purity grade. Especially in case aprotic compounds (such as sulfolane) having a boiling point higher than 20° C above the boiling point of the cyclic acetals is used the formed cyclic acetals can simply be distilled off. In case sulfolane is used as the aprotic compound the formed trioxane can be distilled off without the formation of an azeotrope of sulfolane with trioxane. The process of the invention can be carried out batch wise or as a continuous process.

In a preferred embodiment the process is carried out as a continuous process wherein the formaldehyde source is continuously fed to the liquid medium comprising the catalyst and wherein the cyclic acetals, e.g. the trioxane, is continuously separated (isolated) by separation methods such as distillation.

The process of the invention leads to an extremely high conversion of the formaldehyde source to the desired cyclic acetals.

According to a preferred embodiment the final conversion of the formaldehyde source to the cyclic acetal is greater than 10 %, based on initial formaldehyde source.

The final conversion refers to the conversion of the formaldehyde source into the cyclic acetals in the liquid system. The final conversion corresponds to the maximum conversion achieved in the liquid system.

The final conversion of the formaldehyde source to the cyclic acetals can be calculated by dividing the amount of cyclic acetals (expressed in wt.-%, based on the total weight of the reaction mixture) in the reaction mixture at the

end of the reaction divided by the amount of formaldehyde source (expressed in wt.-%, based on the total weight of the reaction mixture) at the beginning of the reaction at t=0.

For example the final conversion of the formaldehyde source to trioxane can be calculated as:

Final conversion = (amount of trioxane in the reaction mixture expressed in weight-% at the end of the reaction) / (amount of formaldehyde source in the reaction mixture expressed in weight-% at t=0 [initial amount of formaldehyde source in the reaction mixture])

According to a further preferred embodiment of the process of the invention the final conversion of the formaldehyde source into the cyclic acetals, preferably trioxane and/or tetroxane, is higher than 12%, preferably higher than 14%, more preferably higher than 16%, further preferably higher than 20%, especially higher than 30%, particularly higher than 50%, for example higher than 80% or higher than 90%.

According to a further preferred embodiment of the process of the invention the conversion of the formaldehyde source into the cyclic acetals, preferably trioxane and/or tetroxane, is higher than 12%, preferably higher than 14%, more preferably higher than 16%, further preferably higher than 20%, especially higher than 30%, particularly higher than 50%, for example higher than 80% or higher than 90%.

The present disclosure may be better understood with respect to the following example.

Example 1

Anhydrous formaldehyde was prepared by the thermal decomposition of paraformaldehyde (essay: 96 wt%, from Acros Organics) at a rate of ca. 1 g/min at appr. 120 °C and a pressure of 80 mbar. The formaldehyde gas was absorbed in a absorption column containing 500 g sulfolane (<0.1 wt% water) with 0.1 wt% triflic acid at around 40 °C. After 1 hr, the sulfolane in the adsorption column was neutralized with triethylamine and analyzed by GC and sulfite titration. The following composition was found:

Trioxane : 8.3 wt%

Tetroxane: 1.1 wt%

Formaldehyde: 0.6 wt%

Methyl formate: 0.5 wt%

Final conversion of formaldehyde to trioxane in the reaction mixture:

77.5 %

Final conversion of formaldehyde to trioxane and tetroxane in the reaction mixture:

88 %

Example 2

Anhydrous formaldehyde was prepared by the thermal decomposition of paraformaldehyde at a rate of 1 g/min. The formaldehyde gas was absorbed in a absorption column containing 500 g sulfolane (<0.1 wt.-% water) with 0.1 wt.-% trifluoromethanesulfonic acid. The reaction is carried out in a temperature range from 30 to 40 °C. After 50 min the sulfolane in the adsorption column was analysed by gas chromatography (GC) and sulfite titration. The following composition was found:

Trioxane: 6.8 wt%

Tetroxane: 0.9 wt%

Formaldehyde: 1.1 wt%

Methyl formate: 0.7 wt%

Final conversion of formaldehyde to trioxane in the reaction mixture: 71.6 %

Final conversion of formaldehyde to trioxane and tetroxane in the reaction mixture: 81.1 %

## Claims

1. A process for producing oxymethylene homo- or copolymers comprising:

   a) polymerizing at least one monomer to form an oxymethylene polymer in the presence of an initiator;
   b) deactivating the polymerization;
   c) removing residual monomers containing formaldehyde;
   d) contacting the formaldehyde with an aprotic compound having a boiling point of 120 °C or higher determined at 1 bar, and a catalyst; and
   e) at least partly converting the formaldehyde to a cyclic acetal.

2. A process for recovering formaldehyde from an oxymethylene homo- or copolymer production process comprising:

   a) at least partly removing formaldehyde during or after the polymerization process,
   b) contacting said formaldehyde with an aprotic compound having a boiling point of 120 °C or higher determined at 1 bar, and a catalyst; and
   c) at least partly converting the formaldehyde to a cyclic acetal.

3. The process according to claim 1 wherein at least 50 wt.- %, more preferably at least 60 wt.- %, especially at least 75 wt.-%, of the monomer is converted to the oxymethylene homo- or copolymer prior to removing the residual monomers containing formaldehyde and wherein the wt.-% refers to the total weight of the monomers.

4. The process according to claim 1 or 3 wherein the polymerization is deactivated prior to the removal of the residual monomers containing formaldehyde.

5. The process according to claim 2 wherein the formaldehyde is removed as a gaseous mixture together with residual monomers.

6. The process according to one or more of the preceding claims wherein the polymerization process uses trioxane and/or tetroxane as a monomer; and optionally includes a comonomer, and/or
   wherein the formed cyclic acetal is used as a monomer for the oxymethylene homo- or copolymerization process.

7. The process according to one or more of the preceding claims wherein the aprotic compound has a boiling point of 140 °C or higher, more preferably 160 °C or higher, especially 180 °C or higher, determined at 1 bar.

8. The process according to one or more of the preceding claims wherein the formaldehyde, the aprotic compound and the catalyst form a reaction mixture and wherein the reaction mixture comprises at least 20 wt.-%, preferably at least 40 wt.-%, more preferably at least 60 wt.-%, most preferably at least 80 wt.-% and especially at least 90 wt.-% of the aprotic compound, wherein the weight is based on the total weight of the reaction mixture.

9. The process according to one or more of the preceding claims wherein
   the aprotic compound comprises a sulfur containing organic compound, and/or
   wherein the aprotic compound comprises a dipolar nitro-group free compound, and/or
   wherein the aprotic compound is represented by formula (I):

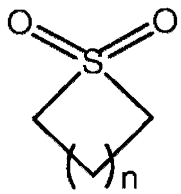

(I)

wherein

n is an integer ranging from 1 to 6, preferably 2 or 3, and
wherein the ring carbon atoms may optionally be substituted by one or more substituents, preferably selected from $C_1$-$C_8$-alkyl which may be branched or unbranched; or
is represented by formula (II):

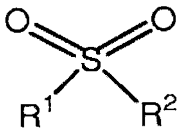

(II)

wherein $R^1$ and $R^2$ are independently selected from $C_1$-$C_8$-alkyl which may be branched or unbranched, preferably wherein $R^1$ and $R^2$ independently represent methyl or ethyl, preferably the aprotic compound is dimethyl sulfone; or is represented by formula (III):

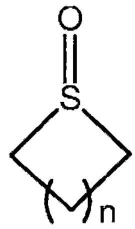

(III)

wherein

n is an integer ranging from 1 to 6, preferably 2 or 3, and
wherein the ring carbon atoms may optionally be substituted by one or more substituents, preferably selected from $C_1$-$C_8$-alkyl which may be branched or unbranched; or
is represented by formula (IV):

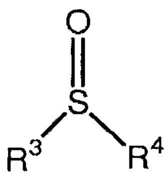

(IV)

wherein $R^3$ and $R^4$ are independently selected from $C_1$-$C_8$-alkyl which may be branched or unbranched, preferably wherein $R^1$ and $R^2$ independently represent methyl or ethyl; preferably the aprotic compound is dimethyl sulfoxide, and/or
wherein the aprotic compound is sulfolane.

**10.** The process according to one or more of the preceding claims wherein the conversion to the cyclic acetal is carried out at a temperature higher than 0°C, preferably ranging from 0°C to 200°C, more preferably ranging from 20°C to 150°C, further preferably from 40°C to 130°C and most preferably from 60°C to 120°C, especially 80°C to 120°C or 80°C to 100°C and is carried out at a pressure of from 10 millibars to 10 bars, and preferably from 0.5 bars to 2 bars, and/or

wherein the removed formaldehyde has a water content of less than 10000 ppm, preferably less than 1000 ppm, most preferably less than 100 ppm.

**11.** The process according to one or more of the preceding claims wherein the residual monomers removed comprise formaldehyde and trioxane, and/or
wherein the initiator comprises boron trifluoride, a heteropoly acid, an isopoly acid, or trifluoromethanesulfonic acid and the catalyst is trifluoromethanesulfonic acid.

**12.** The process according to one or more of the preceding claims, wherein the formaldehyde removed during the process is gaseous formaldehyde and is directly contacted with the aprotic compound and the catalystand/or wherein the removed formaldehyde comprises an aqueous formaldehyde solution when contacted with the aprotic compound and the catalyst.

**13.** An oxymethylene homo- or copolymer obtainable according to one or more of the preceding claims.

**14.** The process according to one or more of the preceding claims, wherein the polymerization process that forms the oxymethylene polymer comprises a heterogeneous process, and/or
wherein the polymerization process that forms the oxymethylene polymer comprises a homogeneous process.

## Patentansprüche

**1.** Verfahren zur Herstellung von Oxymethylenhomo- oder -copolymeren, umfassend:

(a) Polymerisieren mindestens eines Monomers in der Gegenwart eines Initiators, um ein Oxymethylenpolymer zu bilden;
(b) Deaktivieren der Polymerisation;
(c) Entfernen von übrig bleibenden Monomeren enthaltend Formaldehyd;
(d) Inkontaktbringen des Formaldehyds mit einer aprotischen Verbindung mit einem Siedepunkt von 120 °C oder höher, bestimmt bei 1 bar, und einem Katalysator; und
(e) mindestens teilweise Umwandeln des Formaldehyds zu einem cyclischen Acetal.

**2.** Verfahren zur Rückgewinnung von Formaldehyd aus einem Oxymethylenhomo- oder -copolymerherstellungsverfahren, umfassend:

(a) mindestens teilweise Entfernen des Formaldehyds während oder nach dem Polymerisationsverfahren,
(b) Inkontaktbringen des Formaldehyds mit einer aprotischen Verbindung mit einem Siedepunkt von 120 °C oder höher, bestimmt bei 1 bar, und einem Katalysator; und
(c) mindestens teilweise Umwandeln des Formaldehyds zu einem cyclischen Acetal.

**3.** Verfahren nach Anspruch 1, wobei mindestens 50 Gew.-%, stärker bevorzugt mindestens 60 Gew.-%, insbesondere mindestens 75 Gew.-% des Monomers zu dem Oxymethylenhomo- oder -copolymer vor dem Entfernen des übrig bleibenden Monomers, enthaltend Formaldehyd, umgewandelt wird und wobei sich das Gew.-% auf das Gesamtgewicht der Monomere bezieht.

**4.** Verfahren nach Anspruch 1 oder 3, wobei die Polymerisation vor der Entfernung der übrig bleibenden Monomere enthaltend Formaldehyd deaktiviert wird.

**5.** Verfahren nach Anspruch 2, wobei das Formaldehyd als eine gasförmige Mischung zusammen mit den übrig bleibenden Monomeren entfernt wird.

**6.** Verfahren nach einem oder mehreren der vorhergehenden Ansprüche, wobei das Polymerisationsverfahren Trioxan und/oder Tetroxan als Monomer verwendet; und gegebenenfalls ein Comonomer beinhaltet, und/oder wobei das gebildete cyclische Acetal als Monomer für das Oxymethylenhomo- oder -copolymerisationsverfahren verwendet wird.

**7.** Verfahren nach einem oder mehreren der vorhergehenden Ansprüche, wobei die aprotische Verbindung einen Siedepunkt von 140 °C oder höher hat, stärker bevorzugt 160 °C oder höher, insbesondere 180 °C oder höher,

bestimmt bei 1 bar.

**8.** Verfahren nach einem oder mehreren der vorhergehenden Ansprüche, wobei das Formaldehyd, die aprotische Verbindung und der Katalysator eine Reaktionsmischung bilden und wobei die Reaktionsmischung mindestens 20 Gew.-%, vorzugsweise mindestens 40 Gew.-%, stärker bevorzugt mindestens 60 Gew.-%, am stärksten bevorzugt mindestens 80 Gew.-% und insbesondere mindestens 90 Gew.-% der aprotischen Verbindung umfasst, wobei das Gewicht auf dem Gesamtgewicht der Reaktionsmischung basiert.

**9.** Verfahren nach einem oder mehreren der vorhergehenden Ansprüche, wobei die aprotische Verbindung eine Schwefel enthaltende organische Verbindung umfasst, und/oder
wobei die aprotische Verbindung eine dipolare Stickstoffgruppen-freie Verbindung umfasst, und/oder
wobei die aprotische Verbindung durch Formel (I) dargestellt ist:

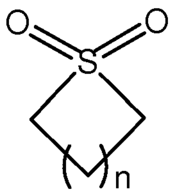

(I)

wobei

n eine ganze Zahl im Bereich von 1 bis 6, vorzugsweise 2 oder 3 ist, und wobei die Kohlenstoffatome des Ringes gegebenenfalls durch einen oder mehrere Substituenten substituiert sein können, vorzugsweise ausgewählt aus $C_1$-$C_8$-Alkyl, das verzweigt oder unverzweigt sein kann; oder dargestellt ist durch Formel (II):

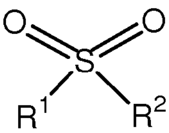

(II)

wobei $R^1$ und $R^2$ unabhängig ausgewählt sind aus $C_1$-$C_8$-Alkyl, das verzweigt oder unverzweigt sein kann, vorzugsweise wobei $R^1$ und $R^2$ unabhängig Methyl oder Ethyl darstellen, vorzugsweise die aprotische Verbindung Dimethylsulfon ist; oder dargestellt ist durch Formel (III):

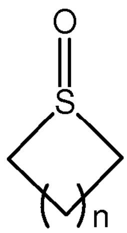

(III)

wobei

n eine ganze Zahl im Bereich von 1 bis 6, vorzugsweise 2 oder 3 ist, und wobei die Kohlenstoffatome des Rings gegebenenfalls durch einen oder mehrere Substituenten substituiert sein können, vorzugsweise ausgewählt aus $C_1$-$C_8$-Alkyl, das verzweigt oder nicht verzweigt sein kann; oder dargestellt ist durch Formel (IV):

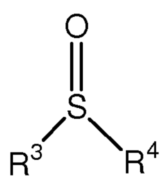

(IV)

wobei $R^3$ und $R^4$ unabhängig ausgewählt sind aus $C_1$-$C_8$-Alkyl, das verzweigt oder unverzweigt sein kann, vorzugsweise wobei $R^1$ und $R^2$ unabhängig Methyl oder Ethyl darstellen; vorzugsweise die aprotische Verbindung Dimethylsulfoxid ist, und/oder
wobei die aprotische Verbindung Sulfolan ist.

**10.** Verfahren nach einem oder mehreren der vorhergehenden Ansprüche, wobei die Umwandlung zu dem cyclischen Acetal bei einer Temperatur höher als 0 °C durchgeführt wird, vorzugsweise im Bereich von 0 °C bis 200 °C, stärker bevorzugt im Bereich von 20 °C bis 150 °C, weiter bevorzugt von 40 °C bis 130 °C und am stärksten bevorzugt von 60 °C bis 120 °C, insbesondere 80 °C bis 120 °C oder 80 °C bis 100 °C, und bei einem Druck von 10 Millibar bis 10 bar und bevorzugt von 0,5 bar bis 2 bar durchgeführt wird, und/oder
wobei das entfernte Formaldehyd einen Wassergehalt von weniger als 10.000 ppm, vorzugsweise weniger als 1.000 ppm, am stärksten bevorzugt weniger als 100 ppm hat.

**11.** Verfahren nach einem oder mehreren der vorhergehenden Ansprüche, wobei die verbleibenden Monomere, die entfernt werden, Formaldehyd und Trioxan umfassen, und/oder
wobei der Initiator Bortrifluorid, eine Heteropolysäure, eine Isopolysäure oder Trifluormethansulfonsäure umfasst und der Katalysator Trifluormethansulfonsäure ist.

**12.** Verfahren nach einem oder mehreren der vorhergehenden Ansprüche, wobei das Formaldehyd, das während des Verfahrens entfernt wird, gasförmiges Formaldehyd ist und direkt mit der aprotischen Verbindung und dem Katalysator in Kontakt gebracht wird, und/oder
wobei das entfernte Formaldehyd eine wässrige Formaldehydlösung umfasst, wenn es mit der aprotischen Verbindung und dem Katalysator in Kontakt gebracht wird.

**13.** Oxymethylenhomo- oder -copolymer, erhältlich nach einem oder mehreren der vorhergehenden Ansprüche.

**14.** Verfahren nach einem oder mehreren der vorhergehenden Ansprüche, wobei das Polymerisationsverfahren, das das Oxymethylenpolymer bildet, ein heterogenes Verfahren umfasst, und/oder
wobei das Polymerisationsverfahren, das das Oxymethylenpolymer bildet, ein homogenes Verfahren umfasst.

## Revendications

**1.** Procédé de production d'homo- ou de copolymères d'oxyméthylène comprenant :

a) la polymérisation d'au moins un monomère pour former un polymère d'oxyméthylène en présence d'un initiateur ;
b) la désactivation de la polymérisation ;
c) l'élimination de monomères résiduels contenant du formaldéhyde ;
d) la mise en contact du formaldéhyde avec un composé aprotique ayant un point d'ébullition supérieur ou égal à 120°C déterminé à 1 bar, et un catalyseur ; et
e) la conversion au moins partielle du formaldéhyde en un acétal cyclique.

**2.** Procédé de récupération de formaldéhyde à partir d'un procédé de production d'homo- ou de copolymère d'oxyméthylène comprenant :

a) l'élimination au moins partielle du formaldéhyde durant ou après le processus de polymérisation,
b) la mise en contact dudit formaldéhyde avec un composé aprotique ayant un point d'ébullition supérieur ou égal à 120°C déterminé à 1 bar, et un catalyseur ; et
c) la conversion au moins partielle du formaldéhyde en un acétal cyclique.

**3.** Procédé selon la revendication 1, dans lequel au moins 50 % en poids, davantage de préférence au moins 60 % en poids, en particulier au moins 75 % en poids, du monomère est converti en l'homo- ou copolymère d'oxyméthylène avant l'élimination des monomères résiduels contenant du formaldéhyde et où le % en poids se rapporte au poids total des monomères.

**4.** Procédé selon la revendication 1 à 3, dans lequel la polymérisation est désactivée avant l'élimination des monomères résiduels contenant du formaldéhyde.

5. Procédé selon la revendication 2, dans lequel le formaldéhyde est éliminé sous forme de mélange gazeux conjointement avec les monomères résiduels.

6. Procédé selon l'une ou plusieurs des revendications précédentes, dans lequel le processus de polymérisation utilise du trioxane et/ou du tétroxane comme monomère ; et éventuellement comprend un comonomère, et/ou
où l'acétal cyclique formé est utilisé comme monomère pour le processus d'homo- ou de copolymérisation d'oxyméthylène.

7. Procédé selon l'une ou plusieurs des revendications précédentes, dans lequel le composé aprotique a un point d'ébullition supérieur ou égal à 140°C, davantage de préférence supérieur ou égal à 160°C, en particulier supérieur ou égal à 180°C, déterminé à 1 bar.

8. Procédé selon l'une ou plusieurs des revendications précédentes, dans lequel le formaldéhyde, le composé aprotique et le catalyseur forment un mélange réactionnel et où le mélange réactionnel comprend au moins 20 % en poids, de préférence au moins 40 % en poids, davantage de préférence au moins 60 % en poids, de préférence entre toutes au moins 80 % en poids et en particulier au moins 90 % en poids du composé aprotique, où le poids est sur la base du poids total du mélange réactionnel.

9. Procédé selon l'une ou plusieurs des revendications précédentes, dans lequel le composé aprotique comprend un composé organique contenant du soufre, et/ou
dans lequel le composé aprotique comprend un composé sans groupe nitro dipolaire, et/ou
dans lequel le composé aprotique est représenté par la formule (I) :

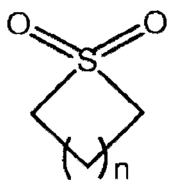

(I)

dans laquelle :

n est un nombre entier se situant dans la plage allant de 1 à 6, de préférence 2 ou 3, et
dans laquelle les atomes de carbone du noyau peuvent éventuellement être substitués par un ou plusieurs substituants, de préférence choisis parmi un groupe alkyle en $C_1$ à $C_8$ qui peut être ramifié ou non ramifié ; ou est représenté par la formule (II) :

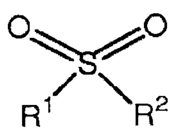

(II)

dans laquelle $R^1$ et $R^2$ sont choisis indépendamment parmi un groupe alkyle en $C_1$ à $C_8$ qui peut être ramifié ou non ramifié, de préférence dans laquelle $R^1$ et $R^2$ représentent indépendamment un groupe méthyle ou éthyle, de préférence le composé aprotique est la diméthylsulfone ; ou est représenté par la formule (III)

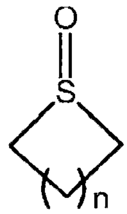

(III)

dans laquelle :

n est un nombre entier se situant dans la plage allant de 1 à 6, de préférence 2 ou 3, et
dans laquelle les atomes de carbone du noyau peuvent éventuellement être substitués par un ou plusieurs

substituants, de préférence choisis parmi un groupe alkyle en $C_1$ à $C_8$ qui peut être ramifié ou non ramifié ; ou est représenté par la formule (IV)

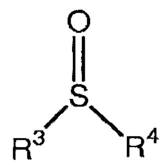

(IV)

dans laquelle $R^3$ et $R^4$ sont choisis indépendamment parmi un groupe alkyle en $C_1$ à $C_8$ qui peut être ramifié ou non ramifié, de préférence dans laquelle $R^1$ et $R^2$ représentent indépendamment un groupe méthyle ou éthyle ; de préférence le composé aprotiue est le diméthylsulfoxyde, et/ou
le composé aprotique étant le sulfolane.

**10.** Procédé selon l'une ou plusieurs des revendications précédentes, dans lequel la conversion en l'acétal cyclique est réalisée à une température supérieure à 0°C, de préférence se situant dans la plage allant de 0°C à 200°C, davantage de préférence se situant dans la plage allant de 20°C à 150°C, encore de préférence de 40°C à 130°C et de préférence entre toutes de 60°C à 120°C, en particulier de 80°C à 120°C ou de 80°C à 100°C et est réalisée à une pression de 10 millibars à 10 bars, et de préférence de 0,5 bars à 2 bars, et/ou
dans lequel le formaldéhyde éliminé a une teneur en eau de moins de 10 000 ppm, de préférence de moins de 1000 ppm, de préférence entre toutes de moins de 100 ppm.

**11.** Procédé selon l'une ou plusieurs des revendications précédentes, dans lequel les monomères résiduels éliminés comprennent le formaldéhyde et le trioxane, et/ou
dans lequel l'initiateur comprend du trifluorure de bore, un hétéropolyacide, un isopolyacide ou de l'acide trifluorométhanesulfonique et le catalyseur est l'acide trifluorométhanesulfonique.

**12.** Procédé selon l'une ou plusieurs des revendications précédentes, dans lequel le formaldéhyde éliminé durant le processus est le formaldéhyde gazeux et est directement mis en contact avec le composé aprotique et le catalyseur et/ou
dans lequel le formaldéhyde éliminé comprend une solution de formaldéhyde aqueuse quand il est mis en contact avec le composé aprotique et le catalyseur.

**13.** Homo- ou copolymère d'oxyméthylène que l'on peut obtenir selon une ou plusieurs des revendications précédentes.

**14.** Procédé selon l'une ou plusieurs des revendications précédentes, dans lequel le processus de polymérisation qui forme le polymère d'oxyméthylène comprend un processus hétérogène, et/ou
dans lequel le processus de polymérisation qui forme le polymère d'oxyméthylène comprend un processus homogène.

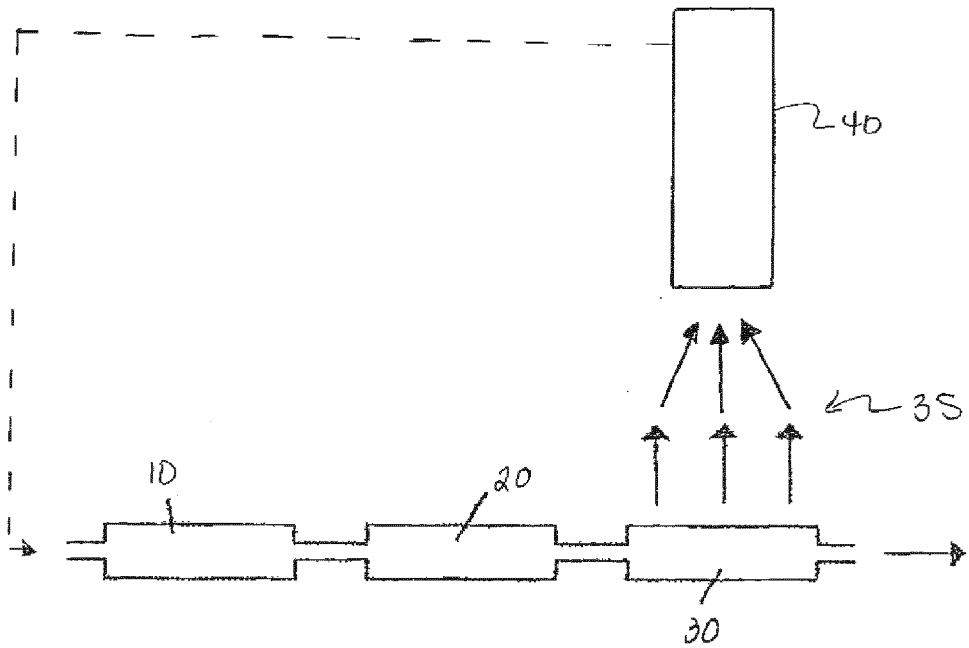


Fig. 1